# EUROPEAN PATENT APPLICATION

(11) **EP 2 899 681 A1**
(43) Date of publication of application: **29.07.2015**
(21) Application number: 15152380.0
(22) Date of filing: 23.01.2015
(51) Int. Cl.: G06Q 40/08

(54) **Method for displaying insurance discount rate and electronic device thereof**

(30) Priority: 24.01.2014 KR 20140008837
(71) Applicant: Samsung Electronics Co., Ltd, Gyeonggi-do 443-742 (KR)
(72) Inventor: Shin, Sang-Min, Gyeonggi-do (KR)
(74) Representative: Jenkins, Richard Gavin

(57) **Abstract**

A method of operating an electronic device is provided. The method includes collecting health-related data of an insurant, transmitting the collected health-related data of the insurant to a server, and receiving an insurance discount rate based on the collected health-related data of the insurant from the server.

## Description

### TECHNICAL FIELD

The present disclosure relates to a method for displaying an insurance discount rate and an electronic device thereof. More particularly, the present disclsoure relates toan apparatus and a method for providing a new health goal level and discount rate to an insurant according to user's health-related data, which varies on a real-time basis, instead of providing a fixed discount rate according to an insurance product.

### BACKGROUND

As gender, age, occupation, and the like, of an insurant are diversified, new insurance products suitable for respective insurants are launched. Accordingly, software development is underway so that an insurance product can be easily selected and managed by interworking with an electronic device which has become a necessity of modem life.

Therefore, a need exists for an apparatus and a method for providing a new health goal level and discount rate to an insurant according to user's health-related data, which varies on a real-time basis, instead of providing a fixed discount rate according to an insurance product.

The above information is presented as background information only to assist with an understanding of the present disclosure. No determination has been made, and no assertion is made, as to whether any of the above might be applicable as prior art with regard to the present disclosure.

### SUMMARY

Aspects of the present disclosure are to address at least the above-mentioned problems and/or disadvantages and to provide at least the advantages described below. Accordingly, an aspect of the present disclosure is to provide an apparatus and a method capable of benefiting both an insurer and an insurant by providing a new health goal level and discount rate according to user's health-related data, which varies on a real-time basis, instead of providing a fixed discount rate according to an insurance product.

Another aspect of the present disclosure is to provide an apparatus and a method capable of improving a user's convenience by automatically collecting health-related data of a user.

Another aspect of the present disclosure is to provide an apparatus and a method capable of motivating a user who intends to achieve a health goal by providing an estimated insurance discount rate according to whether a health goal level is achieved.

In accordance with an aspect of the present disclosure, a method of operating an electronic device is provided. The method includes collecting health-related data of an insurant, transmitting the collected health-related data of the insurant to a server, and receiving an insurance discount rate based on the collected health-related data of the insurant from the server.

In accordance with another aspect of the present disclosure, a method of operating a first server is provided. The method includes calculating a health goal level and an insurance discount rate based on health-related data of an insurant received from at least one electronic device, transmitting data regarding the calculated health goal level and the insurance discount rate to a second server, and receiving data related to the calculated health goal level and insurance discount rate from the second server and transmitting the data to the at least one electronic device.

In accordance with another aspect of the present disclosure, an electronic device is provided. The electronic device includes a communication module and at least one processor configured to collect health-related data of an insurant through the communication module, to transmit the collected health-related data of the insurant to a server, and to receive an insurance discount rate based on the collected health-related data of the insurant from the server.

In accordance with another aspect of the present disclosure, a first server is provided. The first server includes a communication unit, and a controller configured to calculate a health goal level and an insurance discount rate based on health-related data of an insurant received from at least one electronic device, to transmit data regarding the calculated health goal level and the insurance discount rate to a second server, to receive data related to the calculated health goal level and insurance discount rate from the second server, and to transmit the data to the at least one electronic device.

Other aspects, advantages, and salient features of the disclosure will become apparent to those skilled in the art from the following detailed description, which, taken in conjunction with the annexed drawings, discloses various embodiments of the present disclosure.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a block diagram of an electronic device according to an embodiment of the present disclosure;
FIG. 2 is a block diagram of hardware according to an embodiment of the present disclosure;
FIG. 3 is a block diagram of a programming module according to an embodiment of the present disclosure;
FIG. 4 is a block diagram illustrating a structure of a server according to an embodiment of the present disclosure;
FIGS. 5A and 5B illustrate procedures of collecting health-related data in an electronic device according to various embodiments of the present disclosure;
FIG. 6 illustrates a procedure of calculating a health goal level and an insurance discount rate in a first server according to an embodiment of the present disclosure;
FIG. 7 illustrates a procedure of transmitting a determined health goal level and insurance discount rate in a first server according to an embodiment of the present disclosure;
FIGS. 8A, 8B, 8C, and 8D illustrate procedures of displaying an insurance discount rate based on whether a health goal level is achieved in an electronic device according to various embodiments of the present disclosure;
FIGS. 9A, 9B, 9C, and 9D illustrate procedures of additional data provided in an application according to various embodiments of the present disclosure;
FIG. 10 is a flowchart illustrating an operation of an electronic device according to an embodiment of the present disclosure;
FIG. 11 is a flowchart illustrating an operation of a first server according to an embodiment of the present disclosure;
FIG. 12 is a flowchart illustrating a method of an electronic device according to an embodiment of the present disclosure; and
FIG. 13 is a flowchart illustrating a method of a first server according to an embodiment of the present disclosure.

Throughout the drawings, it should be noted that like reference numbers are used to depict the same or similar elements, features, and structures.

### DETAILED DESCRIPTION

The following description with reference to the accompanying drawings is provided to assist in a comprehensive understanding of various embodiments of the present disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the present disclosure .In addition, descriptions of well-known functions and constructions may be omitted for clarity and conciseness.

The terms and words used in the following description and claims are not limited to the bibliographical meanings, but, are merely used by the inventor to enable a clear and consistent understanding of the present disclosure. Accordingly, it should be apparent to those skilled in the art that the following description of various embodiments of the present disclosure is provided for illustration purpose only and not for the purpose of limiting the present disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

An electronic device according to various embodiments of the present disclosure may be a device including a communication function. For example, the electronic device may be one or more combinations of various devices, such as a smart phone, a tablet Personal Computer (PC), a mobile phone, a video phone, an e-book reader, a desktop PC, a laptop PC, a netbook computer, a Personal Digital Assistant (PDA), a Portable Multimedia Player (PMP), a Motion Pictures Expert Group (MPEG-1 or MPEG-2) Audio Layer 3 (MP3) player, a mobile medical device, an electronic bracelet, an electronic necklace, an electronic appcessory, a camera, a wearable device, an electronic clock, a wrist watch, a smart white appliance (e.g., a refrigerator, an air conditioner, a cleaner, an artificial intelligent robot, a TeleVision (TV), a Digital Video Disk (DVD) player, an audio, an oven, a microwave oven, a washing machine, an air purifier, an electronic picture frame, and the like), various medical devices (e.g., Magnetic Resonance Angiography (MRA), Magnetic Resonance Imaging (MRI), Computed Tomography (CR), imaging equipment, ultrasonic instrument, and the like), a navigation device, a Global Positioning System (GPS) receiver, an Event Data Recorder (EDR), a Flight Data Recorder (FDR), a set-top box, a TV box (e.g., Samsung HomeSync^{™}, Apple TV^{™}, or Google TV^{™}), an electronic dictionary, a car infotainment device, an electronic equipment for ship (e.g., a vessel navigation device, a gyro compass, and the like), avionics, a security device, an electronic costume, an electronic key, a camcorder, game consoles, a Head-Mounted Display (HMD), a flat panel display device, an electronic album, a furniture or a part of building/constructions including a communication function, an electronic board, an electronic signature receiving device, a projector, and the like. It is apparent to those ordinarily skilled in the art that the electronic device according to the present disclosure is not limited to the aforementioned devices.

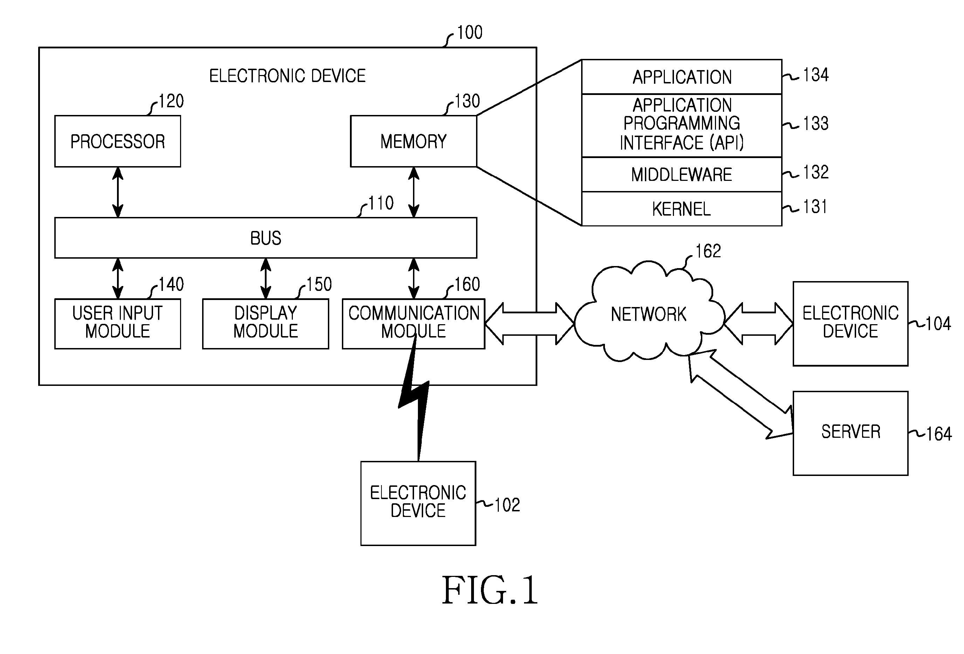

FIG. 1 is a block diagram of an electronic device according to an embodiment of the present disclosure.

Referring to FIG. 1, an electronic device 100 may include a bus 110, a processor 120, a memory 130, a user input module 140, a display module 150, or a communication module 160.

The bus 110 may be a circuit for connecting the aforementioned components and for delivering a communication (e.g., a control message) between the aforementioned components.

The processor 120 may receive an instruction from other components (e.g., the memory 130, the user input module 140, the display module 150, the communication module 160, and the like), for example, via the bus 110, and thus may interpret the received instruction and execute arithmetic or data processing according to the interpreted instruction.

The memory 130 may store an instruction or data received from the processor 120 or other components (e.g., the user input module 140, the display module 150, the communication module 160, and the like) or generated by the processor 120 or other components. The memory 130 may include programming modules, such as a kernel 131, a middleware 132, an Application Programming Interface (API) 133, an application 134, and the like. Each of the aforementioned programming modules may consist of software, firmware, or hardware entities or may consist of at least two or more combinations thereof.

The kernel 131 may control or manage the remaining other programming modules, for example, system resources (e.g., the bus 110, the processor 120, the memory 130, and the like) used to execute an operation or function implemented in the middleware 132, the API 133, or the application 134. In addition, the kernel 131 may provide a controllable or manageable interface by accessing individual components of the electronic device 100 in the middleware 132, the API 133, or the application 134.

The middleware 132 may perform a mediation role so that the API 133 or the application 134 communicates with the kernel 131 to exchange data. In addition, regarding task requests received from the (plurality of) applications 134, for example, the middleware 132 may perform load balancing for the task request by using a method of assigning a priority and the like capable of using a system resource (e.g., the bus 110, the processor 120, the memory 130, and the like) of the electronic device 100 to at least one application among the (plurality of) applications 134.

The API 133 may include, for example, at least one interface or function for file control, window control, video processing, or character control, and the like, as an interface capable of controlling a function provided by the application 134 in the kernel 131 or the middleware 132.

For example, the user input module 140 may receive an instruction or data from a user and deliver it to the processor 120 or the memory 130 via the bus 110. The display module 150 may display video, image, data, and the like, to the user.

The communication module 160 may connect a communication between another electronic device 102 and the electronic device 100. The communication module 160 may support a specific near distance communication protocol (e.g., Wireless Fidelity (WiFi), Bluetooth (BT), Near Field Communication (NFC), or specific network communication 162 (e.g., Internet, Local Area Network (LAN), Wide Area Network (WAN), telecommunication network, cellular network, satellite network, Plain Old Telephone Service (POTS), and the like). Each of the electronic devices 102 and 104 may communicate to a server 164 through the network 162 and may be a device which is the same (e.g., the same type) as the electronic device 100 or may be a different (e.g., a different type) device.

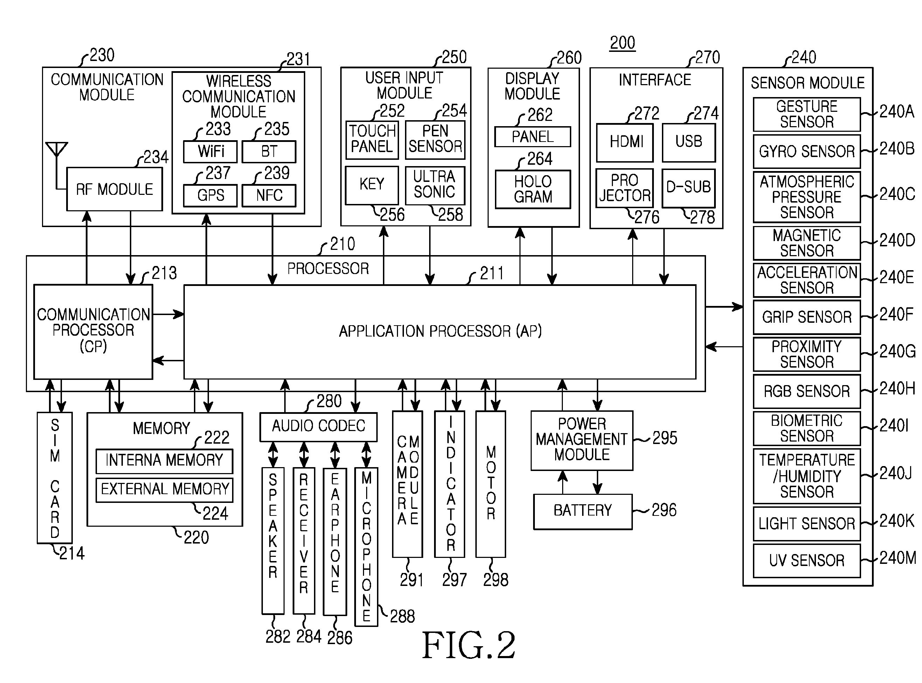

FIG. 2 is a block diagram of hardware according to an embodiment of the present disclosure. For example, hardware 200 may be the electronic device 100 of FIG. 1.

Referring to FIG. 2, the hardware 200 may include one or more processors 210, a Subscriber Identification Module (SIM) card 214, a memory 220, a communication module 230, a sensor module 240, a user input module 250, a display module 260, an interface 270, an audio codec 280, a camera module 291, a power management module 295, a battery 296, an indicator 297, or a motor 298.

The processors 210 (e.g., the aforementioned processor 120) may include one or more Application Processors (APs) 211 or one or more Communication Processors (CPs) 213. For example, the processor 210 may be the processor 120 of FIG. 1. Although it is described in FIG. 2 that the AP 211 and the CP 213 are included in the processor 210, the AP 211 and the CP 213 may be respectively included in different Integrated Circuit (IC) packages. In one embodiment of the present disclosure, the AP 211 and the CP 213 may be included in one IC package. In various embodiments of the present disclosure, the processor 210 may collect health-related data of an insurant. Further, the processor 210 may execute an installed application, calculate an exercise amount by detecting a motion state and a tilt change, and collect data related to the calculated exercise amount. Furthermore, the processor 210 may execute the installed application, perform pairing with a configured second electronic device, calculate an exercise amount by detecting a motion state and tilt change of the second electronic device, and collect data related to the calculated exercise amount.

The AP 211 may control a plurality of hardware or software components connected to the AP 211 by driving an operating system or an application program, and may perform a variety of data processing and computation including multimedia data. For example, the AP 211 may be implemented with a System on Chip (SoC). According to an embodiment of the present disclosure, the processor 210 may further include a Graphic Processing Unit (GPU, not shown).

The CP 213 may perform a function of managing a data link and converting a communication protocol in a communication between other electronic devices connected with an electronic device (e.g., the electronic device 100) including the hardware 200 through a network. For example, the CP 213 may be implemented with an SoC. According to an embodiment of the present disclosure, the CP 213 may perform at least a part of a multimedia control function. For example, the CP 213 may identify and authenticate a terminal in a communication network by using a SIM (e.g., the SIM card 214). In addition, the CP 213 may provide the user with services, such as voice telephony, video telephony, text messages, packet data, and the like.

In addition, the CP 213 may control data transmission/reception of the communication module 230. Although it is illustrated in FIG. 2 that the components, such as the CP 213, the power management module 295, the memory 220, and the like, are separate components independent of the AP 211, according to an embodiment of the present disclosure, the AP 211 may be implemented to include at least a part (e.g., the CP 213) of the aforementioned components.

According to an embodiment of the present disclosure, the AP 211 or the CP 213 may load an instruction or data, received from a non-volatile memory connected thereto or at least one of other components, to a volatile memory and then may process the instruction or data. In addition, the AP 211 or the CP 213 may store data, received from the at least one of other components or generated by the at least one of other components, into the non-volatile memory.

The SIM card 214 may be a card in which a SIM is implemented, and may be inserted to a slot formed at a specific location of the electronic device. The SIM card 214 may include unique identification information (e.g., an IC Card Identifier (ICCID)) or subscriber information (e.g., an International Mobile Subscriber Identity (IMSI)).

The memory 220 may include an internal memory 222 or an external memory 224. For example, the memory 220 may be the memory 130 of FIG. 1. For example, the internal memory 222 may include at least one of a volatile memory (e.g., a Dynamic Random Access Memory (DRAM), a Static RAM (SRAM), a Synchronous DRAM (SDRAM), and the like) or a non-volatile memory (e.g., a One Time Programmable Read-Only Memory (OTPROM), a PROM, an Erasable and Programmable ROM (EPROM), an Electrically Erasable and Programmable ROM (EEPROM), a Mask ROM, a Flash ROM, a NAND flash memory, a NOR flash memory, and the like). According to an embodiment of the present disclosure, the internal memory 222 may have a form of a Solid State Drive (SSD). For example, the external memory 224 may further include Compact Flash (CF), Secure Digital (SD), Micro-SD, Mini-SD, extreme Digital (xD), memory stick, and the like.

The communication module 230 may include a wireless communication module 231 or a Radio Frequency (RF) module 234. For example, the communication module 230 may be the communication module 160 of FIG. 1. For example, the wireless communication module 231 may include a WiFi 233, a BT 235, a GPS 237, or an NFC 239. For example, the wireless communication module 231 may provide a wireless communication function by using a radio frequency. Additionally or alternatively, the wireless communication module 231 may include a network interface (e.g., a LAN card), modem, and the like for connecting the hardware 200 to a network (e.g., Internet, LAN, WAN, telecommunication network, cellular network, satellite network, POTS, and the like). In various embodiments of the present disclosure, the communication module 230 may transmit collected health-related data to a server, and may receive an insurance discount rate based on the collected health-related data from the server. Further, the communication module 230 may receive a new health goal level based on changed health-related data and a new insurance discount rate based thereon from the server. Furthermore, the communication module 230 may receive a health-related message from the server on a real-time basis.

The RF module 234 may serve to transmit/receive data, for example, an RF signal or a paged electronic signal. Although not shown, the RF module 234 may include, for example, a transceiver, a Power Amp Module (PAM), a frequency filter, a Low Noise Amplifier (LNA), and the like. In addition, the RF module 234 may further include a component, e.g., a conductor, a conducting wire, and the like, for transmitting/receiving a radio wave on a free space in a wireless communication.

The sensor module 240 may include, for example, at least one of a gesture sensor 240A, a gyro sensor 240B, a pressure sensor 240C, a magnetic sensor 240D, an acceleration sensor 240E, a grip sensor 240F, a proximity sensor 240G, a Red, Green, Blue (RGB) sensor 240H, a bio sensor 240I, a temperature/humidity sensor 240J, an illumination sensor 240K, and an Ultra Violet (UV) sensor 240M. The sensor module 240 may measure a physical quantity or detect an operation state of the electronic device, and thus may convert the measured or detected information into an electric signal. Additionally/alternatively, the sensor module 240 may include, for example, an E-nose sensor (not shown), an ElectroMyoGraphy (EMG) sensor (not shown), an ElectroEncephaloGram (EEG) sensor (not shown), an ElectroCardioGram (ECG) sensor (not shown), a fingerprint sensor, and the like. The sensor module 240 may further include a control circuit for controlling at least one or more sensors included therein.

The user input module 250 may include a touch panel 252, a (digital) pen sensor 254, a key 256, or an ultrasonic input unit 258. For example, the user input module 250 may be the user input module 140 of FIG. 1. For example, the touch panel 252 may recognize a touch input by using at least one of an electrostatic type, a pressure-sensitive type, an infrared type, and an ultrasonic type. In addition, the touch panel 252 may further include a controller (not shown). In case of the electrostatic type, not only direct touch but also proximity recognition is also possible. The touch penal 252 may further include a tactile layer. In this case, the touch panel 252 may provide the user with a tactile reaction.

For example, the (digital) pen sensor 254 may be implemented by using the same or similar method of receiving a touch input of the user or by using an additional sheet for recognition. For example, the key 256 may be a keypad or a touch key. The ultrasonic input unit 258 is a device by which a terminal detects a sound wave through a microphone (e.g., a microphone 288) by using a pen which generates an ultrasonic signal, and is a device capable of radio recognition. According to an embodiment of the present disclosure, the hardware 200 may use the communication module 230 to receive a user input from an external device (e.g., a network, a computer, or a server) connected thereto.

The display module 260 may include a panel 262 or a hologram 264. For example, the display module 260 may be the display module 150 of FIG. 1. For example, the panel 262 may be a Liquid-Crystal Display (LCD), an Active-Matrix Organic Light-Emitting Diode (AM-OLED), and the like. The panel 262 may be implemented in a flexible, transparent, or wearable manner. The panel 262 may be constructed as one module with the touch panel 252. The hologram 264 may use an interference of light and show a stereoscopic image in the air. According to an embodiment of the present disclosure, the display module 260 may further include a control circuit for controlling the panel 262 or the hologram 264. In various embodiments of the present disclosure, the display module 260 may display data regarding a current health goal level of the insurant, a current discount rate, a discount rate compared with other users, and an expected discount rate.

The interface 270 may include, for example, a High-Definition Multimedia Interface (HDMI) 272, a Universal Serial Bus (USB) 274, a projector 276, or a D-subminiature (D-sub) 278. Additionally or alternatively, the interface 270 may include, for example, SD/Multi-Media Card (MMC) (not shown) or Infrared Data Association (IrDA) (not shown).

The audio codec 280 may bilaterally convert a voice and electronic signal. The audio codec 280 may convert audio information which is input or output, for example, through a speaker 282, a receiver 284, an earphone 286, the microphone 288, and the like.

The camera module 291 is a device for image and video capturing, and according to an embodiment of the present disclosure, may include one or more image sensors (e.g., a front lens or a rear lens), an Image Signal Processor (ISP) (not shown), or a flash LED (not shown).

The power management module 295 may manage power of the hardware 200. Although not shown, the power management module 295 may include, for example, a Power Management IC (PMIC), a charger IC, or a battery fuel gauge.

The PMIC may be equipped, for example, inside an IC or SoC semiconductor. Charging may be classified into wired charging and wireless charging. The charger IC may charge a battery, and may avoid over-voltage or over-current flowing from a charger. According to an embodiment of the present disclosure, the charger IC may further include a charger IC for at least one of the wired charging and the wireless charging. The wireless charging may be classified, for example, into a magnetic resonance type, a magnetic induction type, and an electromagnetic type. An additional circuit for the wireless charging may be added, such as a coil loop, a resonant circuit, a rectifier, and the like.

The battery gauge may measure, for example, a residual quantity of the battery 296 and a voltage, current, and temperature during charging. The battery 296 may generate electricity to supply power source, and for example, may be a rechargeable battery.

The indicator 297 may indicate a specific state, e.g., a booting state, a message state, a charging state, and the like, of the hardware 200 or a part thereof (e.g., the AP 211). The motor 298 may convert an electric signal into a mechanical vibration. A Multipoint Control Unit (MCU)may control the sensor module 240.

Although not shown, the hardware 200 may include a processing unit (e.g., a GPU) for supporting mobile TV. The processing unit for supporting mobile TV may process media data according to a protocol of, for example, Digital Multimedia Broadcasting (DMB), Digital Video Broadcasting (DVB), media flow, and the like.

Names of the aforementioned components of the hardware according to the present disclosure may vary depending on a type of electronic device. The hardware of the present disclosure may include at least one of the aforementioned components. Some of the components may be omitted, or additional other components may be further included. In addition, some of the components of the hardware of the present disclosure may be combined and constructed to one entity, so as to equally perform functions of corresponding components before combination.

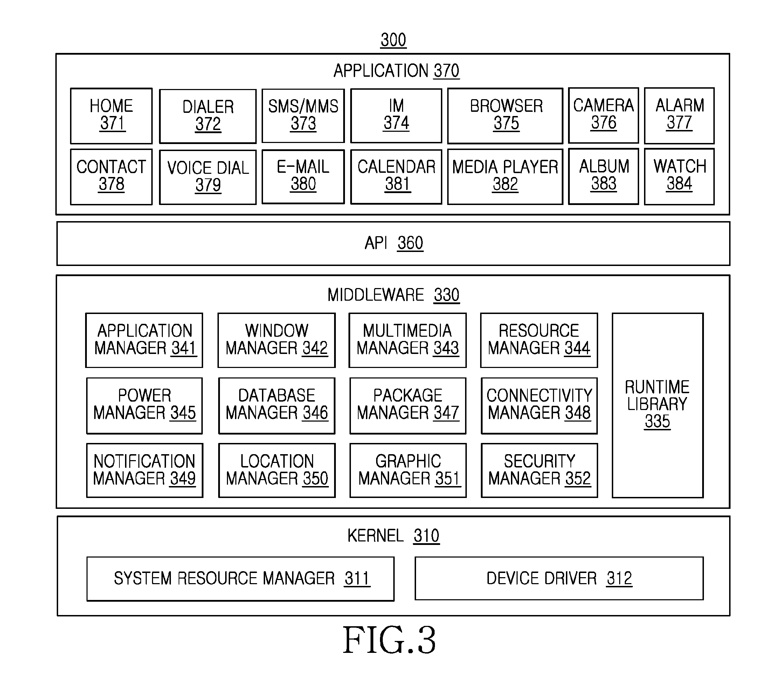

FIG. 3 is a block diagram of a programming module according to an embodiment of the present disclosure. A programming module 300 may be included (e.g., stored) in the electronic device 100 (e.g., the memory 130) of FIG. 1. At least some parts of the programming module 300 may consist of software, firmware, hardware, or a combination of at least two or more of them. The programming module 300 may include an Operating System (OS) implemented in hardware (e.g., the hardware 200) and controlling a resource related to an electronic device (e.g., the electronic device 100) or various applications (e.g., an application 370) driven on the OS. For example, the OS may be Android, iOS, Windows, Symbian, Tizen, Bada, and the like.

Referring to FIG. 3, the programming module 300 may include a kernel 310, a middleware 330, an API 360, or the application 370.

The kernel 310 (e.g., the kernel 131) may include a system resource manager 311 or a device driver 312. The system resource manager 311 may include, for example, a process managing unit, a memory managing unit, a file system managing unit, and the like. The system resource manager 311 may perform control, allocation, retrieval, and the like of the system resource. The device driver 312 may include, for example, a display driver, a camera driver, a BT driver, a shared memory driver, a USB driver, a keypad driver, a WiFi driver, or an audio driver. In addition, the device driver 312 may include an Inter-Process Communication (IPC) driver.

The middleware 330 may include a plurality of modules pre-implemented to provide a function commonly used by the application 370. In addition, the middleware 330 may provide a function through the API 360 so that the application 370 can effectively use a limited system resource in the electronic device. For example, as shown in FIG. 3, the middleware 330 (e.g., the middleware 132) may include at least one of a runtime library 335, an application manager 341, a window manager 342, a multimedia manager 343, a resource manager 344, a power manager 345, a database manager 346, a package manager 347, a connectivity manager 348, a notification manager 349, a location manager 350, a graphic manager 351, and a security manager 352.

The runtime library 335 may include, for example, a library module used by a compiler to add a new function through a programming language while the application 370 is executed. According to an embodiment of the present disclosure, the runtime library 335 may perform an operation of an input/output, a memory management, an arithmetic function, and the like.

The application manager 341 may manage, for example, a life cycle of at least one application among the applications 370. The window manager 342 may manage a Graphic User Interface (GUI) resource used in a screen. The multimedia manager 343 may recognize a format used to reproduce various media files, and may use a code suitable for the format to perform encoding or decoding of the media file. The resource manager 344 may manage a resource (e.g., a source code, a memory, a storage space, and the like) of at least any one of the applications 370.

The power manager 345 may manage a battery or power by operating together with a Basic Input/Output System (BIOS), and the like, and may provide power information, and the like, used for the operation. The database manager 346 may manage to generate, search, or change a database to be used in at least one application among the applications 370. The package manager 347 may manage an installation or update of an application distributed in a form of a package file.

The connectivity manager 348 may manage, for example, a wireless connection, such as WiFi, BT, and the like. The notification manager 349 may display or notify an event, such as an incoming message, an appointment, a proximity notification, and the like, in a manner of not disturbing the user. The location manager 350 may manage location information of the electronic device. The graphic manager 351 may manage a graphic effect to be provided to the user or a user interface related thereto. The security manager 352 may provide a general security function used for system security, user authentication, and the like. According to an embodiment of the present disclosure, if the electronic device (e.g., the electronic device 100) has a telephone function, the middleware 330 may further include a telephony manager (not shown) for managing a voice or video telephony function of the electronic device.

The middleware 330 may generate and use a new middleware module by combining various functions of the aforementioned internal constitutional modules. The middleware 330 may provide a module specified for each type of operating system to provide a differentiated function. In addition, the middleware 330 may dynamically delete some of the existing components or may add new components. Therefore, some of the components described in the various embodiments of the present disclosure may be omitted, or other components may be further included or may be replaced with components having other names for performing a similar function.

The API 360 (e.g., the API 133) is a set of API programming functions, and may be provided with other configurations according to an operating system. For example, in case of Android or iOS, one API set may be provided for each platform, and in case of Tizen, two or more API sets may be provided.

The application 370 (e.g., the application 134) may include, for example, a preloaded application or a third party application. The application 370may include, for example, a home application 371, a dialer application 372, an SMS/MMS application 373, an instant messaging (IM) application 374, a browser application 375, a camera application 376, an alarm application 377, a contact application 378, a voice dial application 379, an E-mail application 380, a calendar application 381, a media player application 382, an album application 383, or a watch application 384.

At least some parts of the programming module 300 may be implemented with an instruction stored in a computer-readable storage media. If it is executed by one or more processors (e.g., the processor 210), the one or more processors may perform a function corresponding to the instruction. For example, the computer-readable storage media may be the memory 260. At least some parts of the programming module 300 may be implemented (e.g., executed), for example, by the processor 210. At least some parts of the programming module 300 may include, for example, modules, programs, routines, sets of instructions, processes, and the like, for performing one or more functions.

Names of components of the programming module (e.g., the programming module 300) according to the present disclosure may vary depending on a type of operating system. In addition, the programming module according to the present disclosure may further include at least one or more components among the aforementioned components, or some of them may be omitted, or additional other components may be further included.

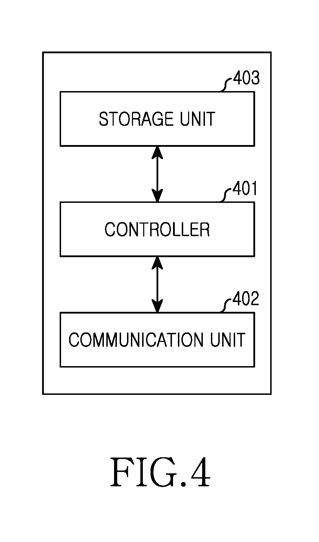

FIG. 4 is a block diagram illustrating a structure of a server according to an embodiment of the present disclosure.

Referring to FIG. 4, the server according to various embodiments of the present disclosure may include a controller 401, a communication unit 402, and a storage unit 403.

First, the controller 401 may control an overall operation of the server. The controller 401 of the server according to various embodiments of the present disclosure may calculate a health goal level and an insurance discount rate based on health-related data received from at least one electronic device. Further, the controller 401 of the server may calculate the health goal level and the insurance discount rate based on received medical data and the health-related data received from the electronic device.

The communication unit 402 processes a signal transmitted/received through an antenna for voice and data communications. The communication unit 402 of the server according to various embodiments of the present disclosure may transmit data related to the calculated health goal level and insurance discount rate to a second server, receive data related to the determined health goal level and insurance discount rate, and transmit the data to at least one electronic device. Further, the communication unit 402 of the server may receive medical data obtained through a health medical examination of a user of the electronic device from the server. Further, the communication unit 402 of the server may receive data regarding a changed health goal level and a changed insurance discount rate from the second server, and may transmit a new health goal level and a new insurance discount rate based thereon to an electronic device in which the health goal level and the insurance discount rate are changed.

The storage unit 403 may consist of a program storage unit which stores a program for controlling an operation of the server and a data storage unit which stores data generated while the program is executed.

In the aforementioned structure, the controller 401 may perform overall functions of the server. However, these functions are separately configured and illustrated in the present disclosure to describe the respective functions separately. Therefore, when the product is implemented in practice, all functions of the server may be processed by the controller 401 or only some of the functions of the portable terminals may be processed by the controller 401.

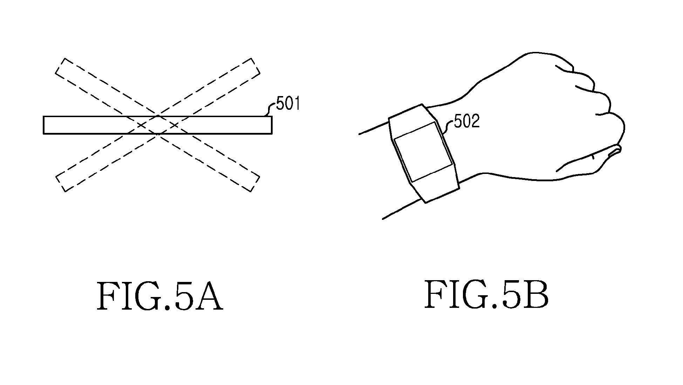

FIGS. 5A and 5B illustrate procedures of collecting health-related data in an electronic device according to various embodiments of the present disclosure. First, the electronic device may execute an application to which an insurance discount rate is applicable by a selection of a user among a plurality of installed applications. For example, the electronic device may execute the selected application capable of providing data related to an insurance discount rate, which varies on a real-time basis, among the plurality of installed applications.

Thereafter, the electronic device may collect health-related data of one or more users according to the selection of the user. More specifically, the electronic device may collect health-related data of the user by receiving a manual input from the user, or may automatically collect the health-related data of the user.

First, a case where the electronic device collects the health-related data when the health-related data is manually input from the user will be described. For example, the electronic device may collect data related to a user's exercise amount based on data related to an exercise time and exercise type, and the like which are input from the user.

For another example, the electronic device may collect data related to a daily calorie intake of the user based on data related to a daily intake of food from the user. For another example, the electronic device may collect data related to a user's health management state based on data related to a user's current blood pressure, blood sugar, and the like received from the user.

Second, the electronic device may automatically collect the health-related data of the user as described above.

Referring to FIG. 5A, an electronic device 501 may detect a motion state, tilt change, and the like of the electronic device 501 in a state where a corresponding application is executed, and thereafter may automatically calculate a user's exercise amount to collect data related to the user's exercise amount.

Referring to FIG. 5B, an electronic device may detect a motion state, a tilt change, and the like of a different electronic device 502 from the different electronic device 502 in which a pairing operation is previously performed, and thereafter may automatically calculate a user's exercise amount to automatically collect data related to the user's exercise amount. Herein, the electronic device detects the motion state, tilt change, and the like of the different electronic device 502 in order to use them when a portability of the electronic device deteriorates. For example, the electronic device may collect data related to a user's exercise amount by performing in advance an operation of pairing with the different electronic device 502 having an easy-to-carry size.

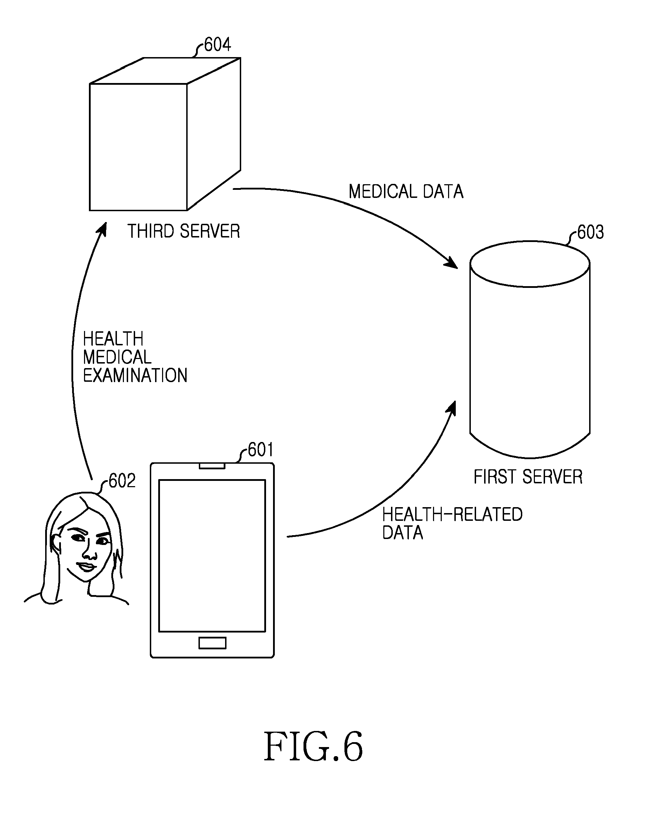

FIG. 6 illustrates a procedure of calculating a health goal level and an insurance discount rate in a first server according to an embodiment of the present disclosure.

Referring to FIG. 6, an electronic device 601 may collect health-related data of one or more users according to a selection of a user 602 in a state where an application capable of confirming the insurance discount rate, which varies on a real-time basis, is executed. More specifically, the electronic device 601 may collect health-related data of the user 602 by receiving a manual input from the user 602, or may automatically collect the health-related data of the user 602.

Thereafter, the electronic device 601 may transmit the collected health-related data of the user 602 to a first server 603. Further, a third server 604 managed by a determined medical center may collect data related to a health medical examination carried out on the user 602 before or after collecting the health-related data in the electronic device 601, and may transmit the data to the first server 603.

Thereafter, the first server 603 may receive health-related data and medical data of the user 602 respectively from the electronic device 601 and the third server 604 managed by the medical center, and may integrally analyze the received data. Herein, the medical data is received from the first server 603 via the third server 604 in the present embodiment to collect more objective health-related data of the user 602.

As described above, the electronic device 601 may manually receive data regarding a blood pressure and blood sugar of the user 602 from the user 602. It is considered in this case that objective data collection is difficult to be achieved. For example, data regarding a health management state of the user 602 in the present embodiment of the present disclosure is collected based on an opinion measured and examined by a determined medical center, and thus an insurance premium can be accurately measured.

Thereafter, based on health-related data received from the electronic device 601 and medical data received from the third server 604, the first server 603 may calculate a health goal level and insurance discount rate of the user 602 of the electronic device 601. For example, in the first server 603, a health goal level and an insurance discount rate for a case of achieving the health goal level to the user 602 may be provided according to insurance terms previously agreed with an insurance company and a current health status, gender, age, and the like, of the user 602.

For example, the present embodiment of the present disclosure is an embodiment in which the first server 603 objectively calculates the health goal level and the insurance discount rate based thereon to be provided to the user 602. Accordingly, if the electronic device 601 manually receives the entirety of health-related data from the user 602, the first server 603 may provide the health goal level and the insurance discount rate for a case where the health goal level is achieved to the user 602 without an aid of the third server 604.

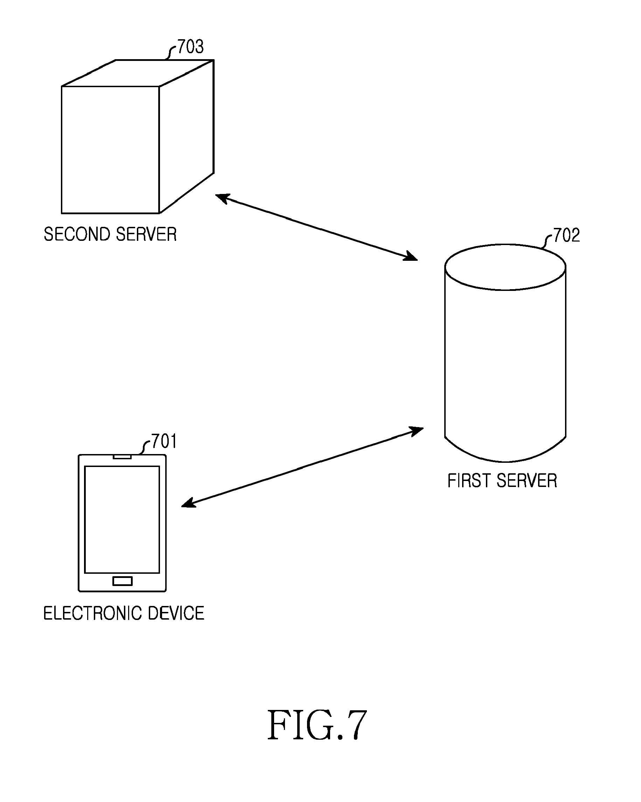

FIG. 7 illustrates a procedure of transmitting a determined health goal level and insurance discount rate in a first server according to an embodiment of the present disclosure.

Referring to FIG. 7, an electronic device 701 may collect health-related data of one or more users according to a selection of a user in a state where an application capable of confirming the insurance discount rate, which varies on a real-time basis, is executed. More specifically, the electronic device 701 may collect health-related data of the user by receiving a manual input from the user, or may automatically collect the health-related data of the user.

Thereafter, if the electronic device 701 may transmit the collected health-related data of the user to a first server 702, the first server 702 may calculate a health goal level and insurance discount rate of the user of the electronic device by integrating the received health-related data and information regarding insurance terms or the like received previously from a second server 703.

Thereafter, the first server 702 may transmit data regarding the calculated health goal level and the insurance discount rate to the second server 703. Herein, the second server 703 may be a server managed by an insurance company with which an insurance contract is made by the user of the electronic device 701.

Thereafter, the second server 703 may receive information indicating whether the health goal level and insurance discount rate received from the first server 702 are approved by an operator of the server. For example, the health goal level and insurance discount rate calculated in the first server 702 and reported to the second server 703 may be approved without alteration by the operator of the server, or may be approved by changing a content thereof.

Thereafter, when the second server 703 transmits again data regarding the determined health goal level and insurance discount rate to the first server 702, the first server 702 may transmit the received data to the electronic device 701.

Thereafter, the electronic device 701 may display information regarding the determined health goal level and insurance discount rate based thereon to the user based on data received from the first server 702.

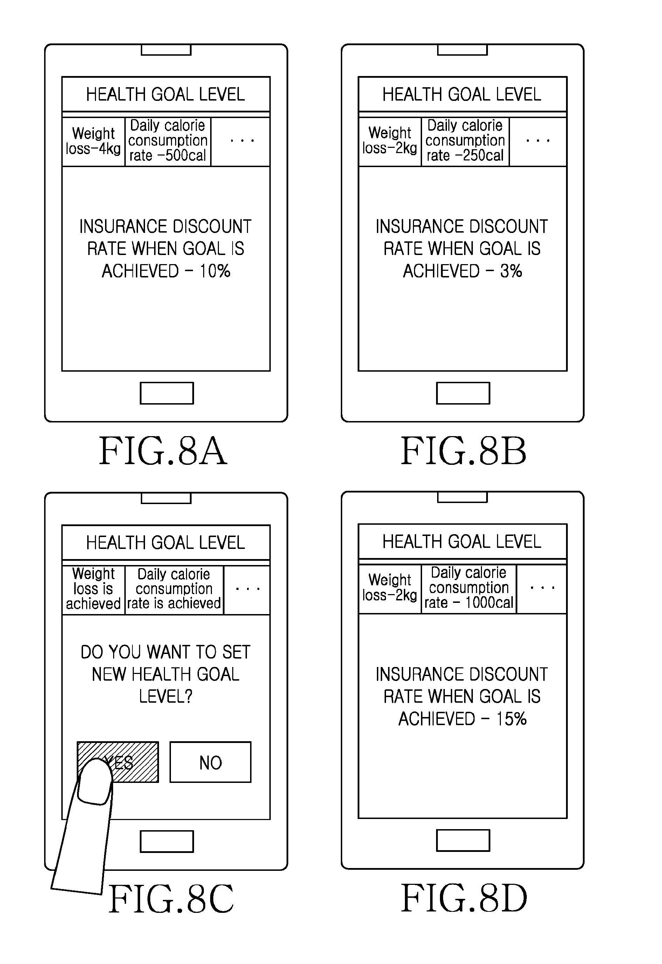

FIGS. 8A, 8B, 8C, and 8D illustrate procedures of displaying an insurance discount rate based on whether a health goal level is achieved in an electronic device according to various embodiments of the present disclosure. First, the electronic device may receive information regarding a determined health goal level and an expected discount rate from a first server and may display the information, based on information regarding a current health status of a user of the electronic device.

Referring to FIG. 8A, the electronic device may display information regarding a user's health goal level, that is, a weight (determined to lose the weight by 4 kilograms), a daily calorie consumption rate (recommended to be lower than 500 calories) to a display module. In addition, the electronic device may display information regarding a specific health goal level and an insurance discount rate (10% discount), which is discounted when the health goal level is achieved, to the display module.

Thereafter, the electronic device may receive a changed health goal level and a discount rate based thereon from the first server according to a user's health status, which varies on a real-time basis.

Referring to FIG. 8B, if the user of the electronic device fails to achieve the health goal level, the electronic device may display an expected insurance discount rate (3%) based on the health goal level (determined to lose the weight by 2 kilograms, and recommended to take a daily calorie consumption rate less than 250 calories) currently achieved by the user and received from the first server.

In the aforementioned example, it can be confirmed that an expected insurance discount rate is decreased from 10% to 3% since the user of the electronic device fails to achieve the health goal level. For example, the health goal level and the expected insurance discount rate according to the present embodiment of the present disclosure are a flexible concept which integrally considers information regarding whether the user of the electronic device achieves the health goal level, a current user's health status, and the like.

Referring to FIGS. 8C and 8D, if the user of the electronic device achieves the health goal level, the electronic device may receive information indicating that the health goal level is achieved and information regarding whether to set a new health goal level from the first server and may display the information.

As shown in FIGS. 8C and 8D, if the electronic device receives from the user an instruction indicating that the new health goal level is set, the electronic device may display information regarding the new health goal level (recommended to lose the weight by 2 kilograms and to take a daily calorie consumption rate less than 1000 calories) and an insurance discount rate (15%) for a case of achieving the goal to a display module.

In the aforementioned example, since the user of the electronic device has already achieved the health goal level, it can be confirmed that an expected insurance discount rate is increased from 10% to 15%. For example, the health goal level and the expected insurance discount rate according to the present embodiment of the present disclosure are a flexible concept which integrally considers information regarding whether the user of the electronic device achieves the health goal level, a current user's health status, and the like.

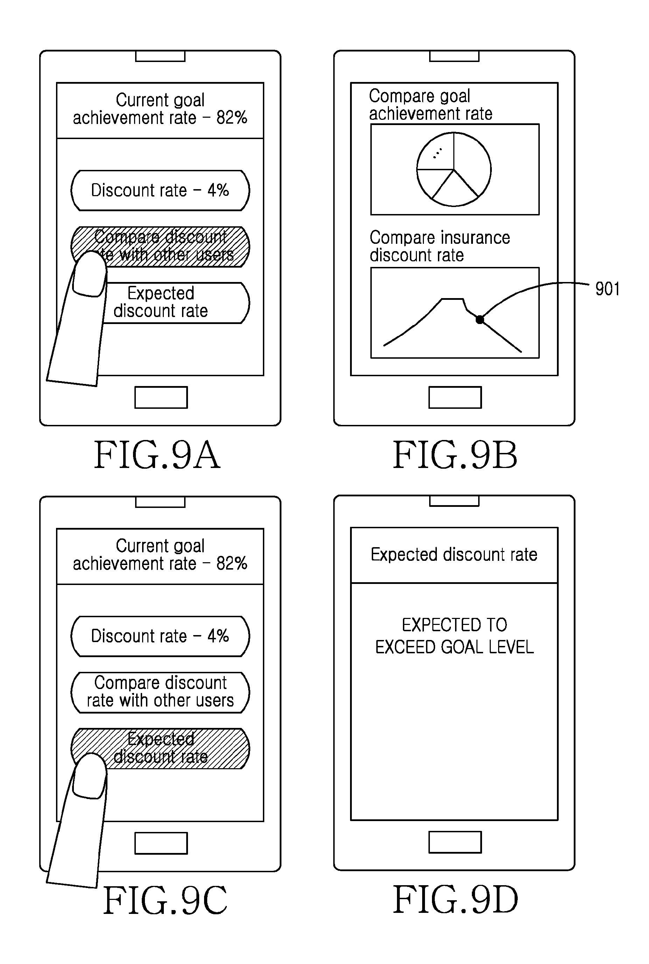

FIGS. 9A, 9B, 9C, and 9D illustrate procedures of additional data provided in an application according to various embodiments of the present disclosure. The application according to various embodiments of the present disclosure may not only automatically collect health-related data of a user but also display additional information together to provide a user convenience.

Referring to FIG. 9A, the application provided in various embodiments of the present disclosure may provide a current goal achievement rate (82%) as well as additional information regarding a discount rate (4%) at a current time point, a discount rate compared with other users, and an expected discount rate.

Referring to FIGS. 9A and 9B, if the electronic device receives an instruction for comparing a discount rate with other users, the electronic device may display a graph for comparing a goal achievement rate (compared according to gender, age, and the like) and a graph 901 for comparing an insurance discount rate (a current position of an insurance discount rate of a user among all insurance subscribers).

Referring to FIGS. 9C and 9D, an application provided in various embodiments of the present disclosure may provide data regarding a current goal achievement rate (82%) and an expected discount rate (expected to exceed a goal level) at a current time according to a selection of a user.

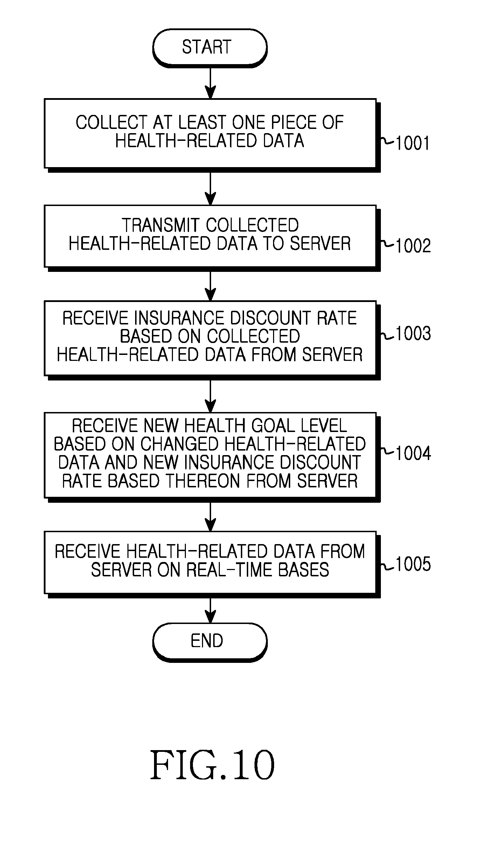

FIG. 10 is a flowchart illustrating an operation of an electronic device according to an embodiment of the present disclosure.

Referring to FIG. 10, the electronic device may collect health-related data of an insurant in operation 1001. More specifically, the electronic device may execute an application to which an insurance discount rate is applicable by a selection of a user among a plurality of installed applications, and thereafter may collect health-related data of the user by receiving a manual input from the user, or may automatically collect the health-related data of the user.

The electronic device may transmit the collected health-related data to the serverin operation 1002. For example, the electronic device may transmit data regarding a user's exercise amount automatically collected to the server.

The electronic device may receive an insurance discount rate based on the collected health-related data from the server in operation 1003. For example, in the electronic device, a health goal level and an insurance discount rate for a case of achieving the health goal level may be provided to the user according to insurance terms previously agreed with an insurance company and a current health status, gender, age, and the like, of the user.

The electronic device may receive a new health goal level based on changed health-related data and a new insurance discount rate based thereon from the server in operation 1004. For example, the health goal level and the expected insurance discount rate according to the present embodiment of the present disclosure are a flexible concept which integrally considers information regarding whether the user of the electronic device achieves the health goal level, a current user's health status, and the like.

The electronic device may receive a health-related message from the server on a real-time basis in operation 1005. For example, the electronic device may receive from the server a numeric value regarding a fine dust in the air in a message form and may display it to the user.

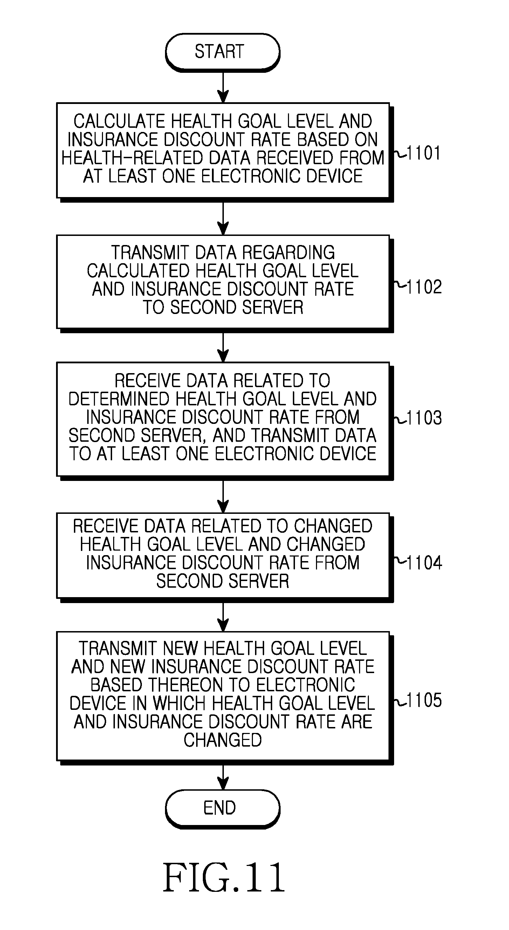

FIG. 11 is a flowchart illustrating an operation of a first server according to an embodiment of the present disclosure.

Referring to FIG. 11, the first server may calculate a health goal level and an insurance discount rate based on health-related data received from at least one electronic device in operation 1101. More specifically, the first server may calculate a health goal level and insurance discount rate of the user of the electronic device by integrating the received health-related data and information regarding insurance terms or the like received previously from a second server.

The first server may transmit data regarding the calculated health goal level and the insurance discount rate to the second server in operation 1102. Herein, the second server may be a server operated by an insurance company with which an insurance contract is made by the user of the electronic device.

The first server may receive data related to the determined health goal level and insurance discount rate from the second server, and may transmit the data to at least one electronic device in operation 1103. For example, the first server may receive data related to the determined health goal level and insurance discount rate from the second server, and may transmit related data to each electronic device.

The first server may receive data related to a changed health goal level and a changed insurance discount rate from the second server in operation 1104. More specifically, the health goal level and the insurance discount rate according to various embodiments of the present disclosure are not fixed values but values which flexibly vary depending on a health status or the like of the user of the electronic device, and thus the first server can receive changed data from the second server.

The first server may transmit a new health goal level and a new insurance discount rate based thereon to the electronic device in which the health goal level and the insurance discount rate are changed in operation 1105. Accordingly, the electronic device which receives new data may display related information to the display module.

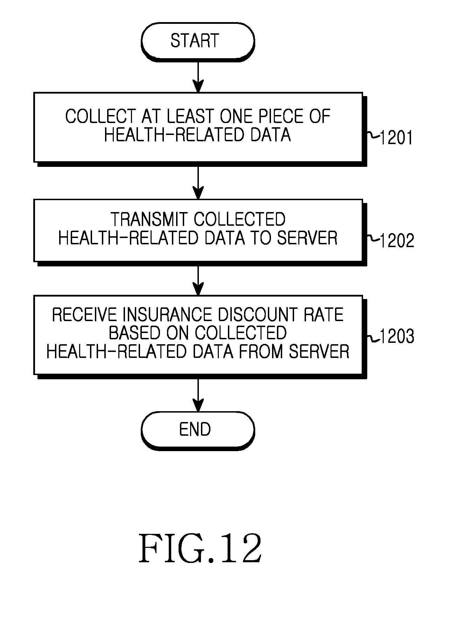

FIG. 12 is a flowchart illustrating a method of an electronic device according to an embodiment of the present disclosure.

Referring to FIG. 12, the electronic device may collect health-related data of an insurant in operation 1201. More specifically, the electronic device may execute an application to which an insurance discount rate is applicable by a selection of a user among a plurality of installed applications, and thereafter may collect health-related data of the user by receiving a manual input from the user, or may automatically collect the health-related data of the user.

The electronic device may transmit the collected health-related data to the server in operation 1202. For example, the electronic device may transmit data regarding a user's exercise amount automatically collected to the server.

The electronic device may receive an insurance discount rate based on the collected health-related data from the server in operation 1203. For example, in the electronic device, a health goal level and an insurance discount rate for a case of achieving the health goal level may be provided to the user according to insurance terms previously agreed with an insurance company and a current health status, gender, age, and the like, of the user.

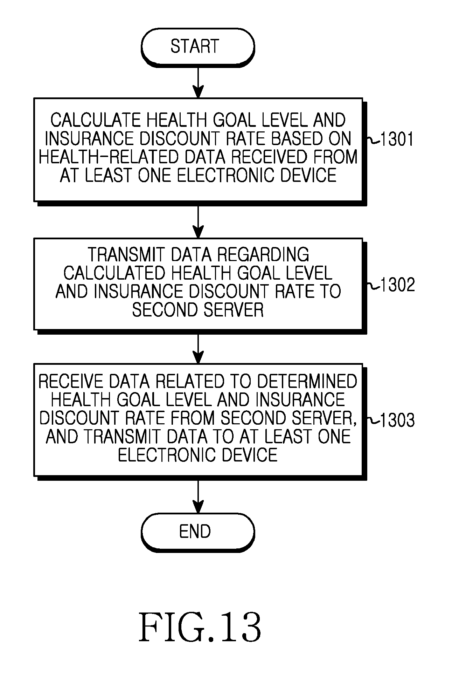

FIG. 13 is a flowchart illustrating a method of a first server according to an embodiment of the present disclosure.

Referring to FIG. 13, the first server may calculate a health goal level and an insurance discount rate based on health-related data received from at least one electronic device in operation 1301. More specifically, the first server may calculate a health goal level and insurance discount rate of the user of the electronic device by integrating the received health-related data and information regarding insurance terms or the like received previously from a second server.

The first server may transmit data regarding the calculated health goal level and the insurance discount rate to the second server in operation 1302. Herein, the second server may be a server operated by an insurance company with which an insurance contract is made by the user of the electronic device.

The first server may receive data related to the determined health goal level and insurance discount rate from the second server, and may transmit the data to at least one electronic device in operation 1303. For example, the first server may receive data related to the determined health goal level and insurance discount rate from the second server, and may transmit related data to each electronic device.

Certain aspects of the present disclosure can also be embodied as computer readable code on a non-transitory computer readable recording medium. A non-transitory computer readable recording medium is any data storage device that can store data which can be thereafter read by a computer system. Examples of the non-transitory computer readable recording medium include a ROM, a RAM, CD-ROMs, magnetic tapes, floppy disks, and optical data storage devices. The non-transitory computer readable recording medium can also be distributed over network coupled computer systems so that the computer readable code is stored and executed in a distributed fashion. In addition, functional programs, code, and code segments for accomplishing the present disclosure can be easily construed by programmers skilled in the art to which the present disclosure pertains.

At this point it should be noted that the various embodiments of the present disclosure as described above typically involve the processing of input data and the generation of output data to some extent. This input data processing and output data generation may be implemented in hardware or software in combination with hardware. For example, specific electronic components may be employed in a mobile device or similar or related circuitry for implementing the functions associated with the various embodiments of the present disclosure as described above. Alternatively, one or more processors operating in accordance with stored instructions may implement the functions associated with the various embodiments of the present disclosure as described above. If such is the case, it is within the scope of the present disclosure that such instructions may be stored on one or more non-transitory processor readable mediums. Examples of the processor readable mediums include a ROM, a RAM, CD-ROMs, magnetic tapes, floppy disks, and optical data storage devices. The processor readable mediums can also be distributed over network coupled computer systems so that the instructions are stored and executed in a distributed fashion. In addition, functional computer programs, instructions, and instruction segments for accomplishing the present disclosure can be easily construed by programmers skilled in the art to which the present disclosure pertains.

While the present disclosure has been shown and described with reference to various embodiments thereof, it will be understood by those skilled in the art that various changes in form and details may be made therein without departing from the spirit and scope of the present disclosure as defined by the appended claimsand their equivalents.

## Claims

1. A method of operating an electronic device, the method comprising:
collecting health-related data of an insurant;
transmitting the collected health-related data of the insurant to a server; and
receiving an insurance discount rate based on the collected health-related data of the insurant from the server.

2. The method of claim 1, wherein the collecting of the health-related data of the insurant comprises:
executing an application;
calculating an exercise amount by detecting a motion state and a tilt change of the electronic device; and
collecting data related to the calculated exercise amount.

3. The method of claim 1, wherein the collecting of the health-related data of the insurant comprises:
executing an application;
performing paring with a configured different electronic device;
calculating an exercise amount by detecting a motion state and tilt change of the different electronic device; and
collecting data related to the calculated exercise amount.

4. The method of claim 1, wherein the insurance discount rate comprises an insurance discount rate dependent on a flexible health goal level based on the collected health-related data of the insurant.

5. The method of claim 1, further comprising receiving a new health goal level based on changed health-related data and a new insurance discount rate based thereon from the server.

6. The method of claim 1, further comprising:
executing an application; and
displaying data regarding a current health goal level of the insurant, a current discount rate, a discount rate compared with other insurance subscribers, and an expected discount rate.

7. The method of claim 1, further comprising receiving a health-related message from the server on a real-time basis.

8. A method of operating a first server, the method comprising:
calculating a health goal level and an insurance discount rate based on health-related data of an insurant received from at least one electronic device;
transmitting data regarding the calculated health goal level and the insurance discount rate to a second server; and
receiving data related to the calculated health goal level and insurance discount rate from the second server and transmitting the data to the at least one electronic device.

9. The method of claim 8, wherein the calculating of the health goal level and the insurance discount rate comprises:
receiving medical data obtained through a health medical examination of a user of the at least one electronic device from a third server; and
calculating the health goal level and the insurance discount rate based on the received medical data and the health-related data of the insurant received from the at least one electronic device.

10. The method of claim8, further comprising:
receiving data regarding a changed health goal level and a changed insurance discount rate from the second server; and
transmitting a new health goal level and a new insurance discount rate based thereon to the at least one electronic device in which the health goal level and the insurance discount rate are changed.

11. An electronic device comprising:
a communication module; and
at least one processor configured:
to collect health-related data of an insurant through the communication module,
to transmit the collected health-related data of the insurant to a server, and
to receive an insurance discount rate based on the collected health-related data of the insurant from the server.

12. The electronic device of claim 11, wherein the at least one processor is further configured:
to execute an application,
to calculate an exercise amount by detecting a motion state and a tilt change of the electronic device, and
to collect data related to the calculated exercise amount.

13. The electronic device of claim 11, wherein the at least one processor is further configured:
to execute an application,
to perform paring with a configured different electronic device,
to calculate an exercise amount by detecting a motion state and tilt change of the different electronic device, and
to collect data related to the calculated exercise amount.

14. The electronic device of claim 11, wherein the insurance discount rate comprises an insurance discount rate dependent on a flexible health goal level based on the collected health-related data of the insurant.

15. The electronic device of claim 11,wherein the at least one processor is further configured to receive a new health goal level based on a changed health-related data and a new insurance discount rate based thereon from the server.
